# EUROPEAN PATENT APPLICATION

(11) **EP 4 563 129 A1**
(43) Date of publication of application: **04.06.2025**
(21) Application number: 23846798.9
(22) Date of filing: 09.06.2023
(51) Int. Cl.: A61F 9/007, A61F 2/14

(54) **IMPLANT DEVICE HAVING MULTIPLE CHANNELS FOR EYE DISEASES, AND METHOD FOR MANUFACTURING SAME**

(30) Priority: 25.07.2022 KR 20220091662
(71) Applicant: Microt Inc., Seoul 06351 (KR)
(72) Inventor: RYU, Junoh, Gwacheon-si Gyeonggi-do 13830 (KR)
(74) Representative: Botti & Ferrari S.p.A.
(86) International application number: PCT/KR2023/007932
(87) International publication number: WO 2024/025138

(57) **Abstract**

An implant device for an eye disease to be inserted into an eyeball may include: a plurality of tubes each having one end to be inserted into an anterior chamber of the eyeball and having a hollow portion through which aqueous humor is drained; and a connector configured to couple the plurality of tubes. The connector is coupled to the plurality of tubes so that the plurality of tubes extend through the connector. In this case, the plurality of tubes are different from each other in a length, a material, a diameter of the hollow portion, whether a ripcord is inserted into a tube, and a diameter and/or a material of the ripcord inserted into the tube, and thus, are different from each other in aqueous humor drainage characteristics such as the amount of aqueous humor drainage and a drainage rate through the tubes and/or an aqueous humor drainage position through the tubes.

## Description

### [Technical Field]

The present disclosure relates to an implant device for an eye disease and a method of manufacturing the same, and more particularly, to an implant device for an eye disease configured to prevent loss of an aqueous humor drainage function due to fibrosis or foreign material mixing after implant surgery by connecting a plurality of tubes having different aqueous humor drainage characteristics (drainage amount, drainage rate, etc.) and/or different drainage positions through a connector.

### [Background Art]

For a glaucoma patient whose intraocular pressure is not controlled even by using an intraocular pressure lowering agent, intraocular pressure is lowered by creating a bypass to drain aqueous humor from an anterior chamber of an eye to an external surface of the eye under the conjunctiva. Trabeculectomy among glaucoma filtration surgeries that create a bypass or a fistula for aqueous humor drainage may fail to control intraocular pressure when the amount of aqueous humor drainage decreases due to closure of the bypass after surgery. When initial surgery fails and glaucoma filtration surgery is performed again, the frequency of bypass closure after surgery increases and a success rate of surgery is low.

Also, even in the case of intractable glaucoma such as neovascular glaucoma or secondary glaucoma caused by uveitis according to types of glaucoma, closure of a bypass frequently occurs after trabeculectomy, resulting in poor results. For an eye with a history of failed glaucoma filtration surgery or intractable glaucoma, glaucoma implant surgery of placing a glaucoma implant device is performed to prevent closure of a bypass and increase a success rate of surgery. To date, glaucoma implant surgery has been used as an alternative to trabeculectomy, especially in several difficult-to-treat glaucoma, in that glaucoma implant surgery not only effectively lowers intraocular pressure but also shows a predictable postoperative clinical outcome according to an inner diameter of a given tube.

However, an existing glaucoma implant used for glaucoma implant surgery may cause various problems and complications such as difficulty in surgery due to a relatively large size, postoperative exposure, infection, eye movement disorder due to a large body, and diplopia. Accordingly, small-sized glaucoma implant instruments for minimally-invasive glaucoma surgery (MIGS) have recently been developed to relatively easily lower intraocular pressure by using a glaucoma implant and to reduce side effects after surgery due to a large size.

MIGS is a method of inserting a tube with a size of micrometers into an anterior chamber of an eye to drain aqueous humor from the anterior chamber. There is a possibility that the tube may become blocked due to fibrosis of tissue surrounding the eye or a foreign material at an aqueous humor outlet of the tube. In particular, a degree of intraocular pressure control through an implant device varies greatly according to a postoperative period, fibrosis of the tube, and foreign material mixing. For example, immediately after implant surgery, a significant amount of aqueous humor is drained through the tube, causing intraocular pressure to be lowered more than necessary. However, several weeks after the surgery, an area around an aqueous humor outlet of the tube may become fibrotic, gradually reducing the amount of aqueous humor drained through the tube. When several months have passed since the surgery in this state, most of the area around the aqueous humor outlet may become fibrotic, making it difficult to drain the aqueous humor through the tube and drastically reducing the effectiveness of the implant surgery. Also, sometimes, a foreign material may be mixed into the tube, weakening the aqueous humor drainage function.

### [Disclosure]

### [Technical Problem]

According to one aspect of the present disclosure, the problems of the related art may be solved, and there may be provided an implant device for an eye disease in which the amount of aqueous humor drainage may be appropriately maintained even when fibrosis of surrounding tissue progresses or a foreign material is mixed into a tube after implant surgery, and a method of manufacturing the implant device for an eye disease.

Also, according to one aspect of the present disclosure, there may be provided an implant device for an eye disease in which a cross-section of a connector for coupling tubes of the implant device to each other is at least partially larger than a diameter of each of the tubes, so that when the tube is pushed into an eyeball after implant surgery, the connector is caught on a sclera of the eyeball and the tube is prevented from being completely inserted into the sclera.

### [Technical Solution]

According to an embodiment of the present disclosure, an implant device for an eye disease to be inserted into an eyeball includes: a plurality of tubes each having one end to be inserted into an anterior chamber of the eyeball and having a hollow portion through which aqueous humor is drained; and a connector configured to couple the plurality of tubes. In this case, the connector is coupled to the plurality of tubes so that the plurality of tubes extend through the connector.

In an embodiment, the plurality of tubes are different from each other in at least one of a length, a material, and a diameter of a hollow portion, and thus are different from each other in at least one of aqueous humor drainage characteristics or an aqueous humor drainage position.

In an embodiment, each of the plurality of tubes includes a first end to be inserted into the anterior chamber of the eyeball and a second end opposite to the first end, wherein the second ends of the plurality of tubes are located at different positions from the connector.

The implant device for an eye disease according to an embodiment further includes a ripcord inserted into the hollow portions of some of the plurality of tubes. The implant device for an eye disease according to another embodiment further includes a plurality of ripcords respectively inserted into the hollow portions of the plurality of tubes, wherein the plurality of ripcords are different from each other in at least one of a diameter and a material.

In an embodiment, the connector includes a plurality of holes through which the plurality of tubes respectively pass, the plurality of holes being spaced apart from each other in a cross-section of the connector. In another embodiment, the connector includes a hole extending in a direction orthogonal to a longitudinal direction of the plurality of tubes so that the plurality of tubes pass through the hole.

In an embodiment, the connector has a cross-section larger than a width of the plurality of tubes.

In an embodiment, the connector includes: a first portion through which the plurality of tubes pass; and a second portion extending from the first portion in a direction different from an extending direction of the plurality of tubes, the second portion protruding in a lateral direction of the plurality of tubes. In this case, a thickness of the second portion may be less than a thickness of the first portion.

In an embodiment, the plurality of tubes are integrally formed with each other. Also, in an embodiment, the plurality of tubes and the connector are integrally formed with each other.

According to an embodiment of the present disclosure, a method of manufacturing an implant device for an eye disease includes: forming a plurality of tubes each having a hollow portion through which aqueous humor is drained; forming a body of a connector extending in one direction; forming, in the body, one or multiple holes through which the plurality of tubes pass; and inserting the plurality of tubes into the hole of the body. In this case, the plurality of tubes are different from each other in at least one of aqueous humor drainage characteristics or an aqueous humor drainage position.

According to still another embodiment of the present disclosure, a method of manufacturing an implant device for an eye disease includes forming a tube body including a plurality of tubes each having a hollow portion through which aqueous humor is drained and a connector integrally connected to the plurality of tubes. In this case, the connector extends in one direction and is coupled to the plurality of tubes so that the plurality of tubes extend in a direction different from a longitudinal direction of the connector, wherein the plurality of tubes are different from each other in at least one of aqueous humor drainage characteristics or an aqueous humor drainage position.

### [Advantageous Effects]

According to an embodiment of the present disclosure, in an implant device for an eye disease, because a plurality of tubes different in terms of length, material, diameter of a hollow portion, whether a ripcord is inserted into a tube, and diameter and/or material of the ripcord inserted into the tube and thus different in terms of aqueous humor drainage characteristics (drainage amount, drainage rate, etc.) and/or drainage positions are connected to a connector, an aqueous humor outlet of all of the tubes may be prevented from being blocked due to fibrosis of surrounding tissue or foreign material mixing after implant surgery.

Also, in the implant device for an eye disease according to an embodiment of the present disclosure, because a cross-section of the connector is at least partially larger than a diameter of each of the tubes, even when the tube is pushed into an eyeball due to any cause after surgery using the implant device, the connector with a relatively large cross-section may be caught on a sclera of the eyeball and the tube may be prevented from being completely inserted into the sclera of the eyeball.

### [Description of Drawings]

FIGS. 1 and 2 are conceptual views illustrating a state where an implant device for an eye disease according to an embodiment is inserted into an eyeball.
FIG. 3A is a perspective view illustrating an implant device for an eye disease according to an embodiment.
FIG. 3B is a cross-sectional view illustrating the implant device for an eye disease of FIG. 3A.
FIG. 4 is a perspective view illustrating an implant device for an eye disease according to another embodiment.
FIG. 5A is a perspective view illustrating an implant device for an eye disease according to still another embodiment.
FIG. 5B is a cross-sectional view illustrating the implant device for an eye disease of FIG. 5A.
FIGS. 6A and 6B are perspective views illustrating an implant device for an eye disease according to still other embodiments.
FIG. 7A is a conceptual view for describing a process of manufacturing an implant device for an eye disease according to an embodiment.
FIG. 7B is a perspective view illustrating an implant device for an eye disease that is integrally formed according to still another embodiment.
FIG. 8A is a perspective view illustrating an implant device for an eye disease according to still another embodiment.
FIG. 8B is a cross-sectional view illustrating the implant device for an eye disease of FIG. 8A.
FIG. 9 is cross-sectional views illustrating various cross-sectional shapes of a connector in an implant device for an eye disease according to embodiments.
FIG. 10 is a conceptual view illustrating a process of inserting an implant device for an eye disease according to an embodiment into an eyeball by using an injector.

### [Best Mode]

The terms used herein will be briefly described, and the present disclosure will be described in detail.

The terms used herein are general terms currently widely used in the art in consideration of functions in the present disclosure, but the terms may vary according to the intention of one of ordinary skill in the art, precedents, or new technology in the art. Also, some of the terms used herein may be arbitrarily chosen by the present applicant, and in this case, these terms are defined in detail below. Accordingly, the specific terms used herein should be defined based on the unique meanings thereof and the whole context of the present disclosure.

It will be understood that when a certain part "includes" a certain component, the part does not exclude another component but may further include another component, unless the context clearly dictates otherwise. Also, throughout the specification, when an element is referred to as being "connected" to another element, it will be understood to include that the element is "directly connected" to the other element or is "connected" to the other element with another element therebetween.

The present disclosure will now be described more fully with reference to the accompanying drawings for one of ordinary skill in the art to be able to perform the present disclosure without any difficulty. However, the present disclosure may be embodied in many different forms and is not limited to the embodiments set forth herein. For clarity, portions irrelevant to the descriptions of the present disclosure are omitted in the drawings, and like components are denoted by like reference numerals throughout the specification.

Hereinafter, the present disclosure will be described in detail with reference to the accompanying drawings.

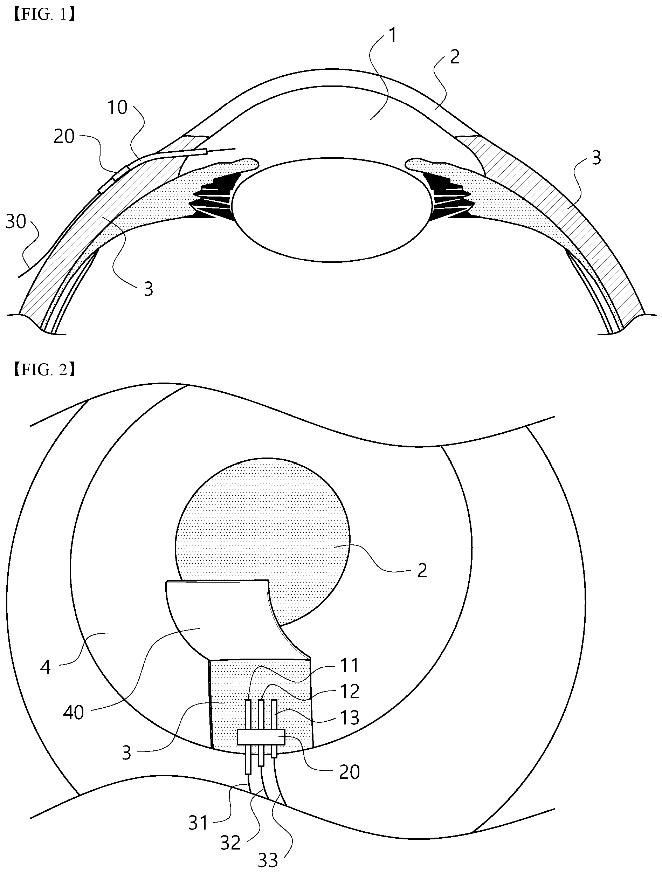

FIGS. 1 and 2 are conceptual views illustrating a state where an implant device for an eye disease according to an embodiment is inserted into an eyeball

Referring to FIGS. 1 and 2, the implant device according to embodiments is intended to control intraocular pressure by controlling the amount of aqueous humor drainage in an anterior chamber 1 located in front of a lens and below a cornea 2 in an eyeball, thereby preventing damage to an optic nerve due to increased intraocular pressure caused by eye diseases. The implant device for an eye disease according to embodiments of the present disclosure may be used to treat or alleviate symptoms of various eye diseases that cause or are caused by increased intraocular pressure.

Eye diseases in the specification may include glaucoma caused by an increase in intraocular pressure. Examples of glaucoma may include, but are not limited to, congenital glaucoma, traumatic glaucoma, glaucoma suspect, ocular hypertension, primary open-angle glaucoma, normal-tension glaucoma, capsular glaucoma with pseudoexfoliation of lens, chronic simple glaucoma, low-tension glaucoma, pigmentary glaucoma, primary angle-closure glaucoma, acute angle-closure glaucoma, chronic angle-closure glaucoma, intermittent angle-closure glaucoma, glaucoma secondary to eye trauma, glaucoma secondary to eye inflammation, glaucoma secondary to drugs, neovascular glaucoma, and secondary glaucoma due to uveitis.

The implant device for an eye disease according to embodiments may include a tube 10 applicable to minimally-invasive glaucoma surgery (MIGS), and one end of the tube 10 may be inserted into the anterior chamber 1 of the eyeball and the other end of the tube 10 may be located on conjunctival tissue or Tenon's tissue 4. The tube 10 may include a hollow portion through which aqueous humor of the eyeball may flow, and may allow the aqueous humor to be drained from the anterior chamber 1 of the eyeball to the outside of a sclera 3 through the tube 10. In an embodiment, a ripcord 30 having a function of adjusting the amount of aqueous humor drainage through the hollow portion of the tube 10 may be inserted into the tube 10.

Referring to FIG. 2, the implant device for an eye disease may be inserted after an operator exfoliates the conjunctival tissue or Tenon's tissue 4 of the eyeball, and after insertion, the implant device may be placed in the eyeball by covering the conjunctival tissue or Tenon's tissue 4 again. That is, after a flap 40 is created by cutting a part of the conjunctival tissue or Tenon's tissue 4, the created flap 40 may be lifted and distal ends of tubes 11-13 may be inserted to pass through the exposed sclera 3 of the eyeball. In this case, in the implant device for an eye disease according to embodiments, the plurality of tubes 11-13 are coupled to each other by one connector 20. Also, each of the plurality of tubes 11-13 may not be completely inserted into the sclera 3, and a proximal end of each of the tubes 11-13 may be placed on or inserted into the conjunctival tissue or Tenon's tissue 4. After one end of each of the tubes 11-13 is inserted into the anterior chamber 1 through the sclera 3, a process of inserting the implant device for an eye disease is completed by putting down the lifted flap 40.

In the implant device for an eye disease according to embodiments, the plurality of tubes 11-13 are configured to be different from each other in terms of aqueous humor drainage characteristics, for example, the amount of aqueous humor drainage and a drainage rate. In the implant device, because the tubes 11-13 having different aqueous humor drainage characteristics are coupled to each other by the connector 20, when fibrosis of surrounding tissue or foreign material mixing occurs after implant surgery, an aqueous humor outlet through all of the tubes 11-13 may be prevented from being blocked. In order to have different aqueous humor drainage characteristics, the plurality of tubes 11-13 may be configured to have different lengths, materials, and/or diameters of hollow portions.

In an embodiment, the implant device for an eye disease further includes ripcords 31-33 inserted into the hollow portions of the tubes 11-13. The ripcords 31-33 are for controlling pressure formed in the anterior chamber through the hollow portions of the tubes 11-13. When the ripcords 31-33 are inserted into the hollow portions of the tubes 11-13, the flow of the aqueous humor becomes unsmooth, causing the aqueous humor to be accumulated in the anterior chamber of the eye, and intraocular pressure is relatively increased compared to when the ripcords 31-33 are not present. In the specification, pressure formed in the anterior chamber refers to pressure in the anterior chamber of the eyeball formed at this time. The implant device for an eye disease may be inserted into the eyeball with the ripcords 31-33 inserted into the tubes 11-13, or the implant device for an eye disease, excluding the ripcords 31-33, may be inserted into the eye and then the ripcords 31-33 may be inserted into the tubes 11-13.

Although the ripcords 31-33 are respectively inserted into all of the plurality of tubes 11-13 constituting the implant device in FIG. 2, this is only an example, and in another embodiment, ripcords may be inserted into only some of the plurality of tubes 11-13 so that the plurality of tubes 11-13 have different aqueous humor drainage characteristics. Also, even among the tubes into which the ripcords are inserted, diameters and/or materials of the ripcords inserted into the tubes may be different from each other so that the plurality of tubes have different aqueous humor drainage characteristics.

Also, in an embodiment, in the implant device for an eye disease, an implant body (not shown) may be further coupled to the proximal ends of the tubes 11-13 located on the conjunctival tissue or Tenon's tissue 4 of the eyeball. The implant body may include one or more membranes arranged to surround ends of the tubes 11-13, and each membrane may be formed of, for example, a urethane-based material such as polytetrafluoroethylene (PTFE) or polycarbonate polyurethane, a silicone-based material such as polydimethylsiloxane (PDMS), a siloxane/polyurethane compound (siloxane-based polyurethane), polyethylene, polypropylene, or polymethyl methacrylate (PMMA).

The implant body is coupled to the rear of the tubes 11-13 and temporarily accommodates the aqueous humor to effectively control intraocular pressure. For example, the implant body may be coupled and disposed at the rear of the tubes 11-13 through the exfoliated conjunctival tissue or Tenon's tissue 4 in consideration of a clinical situation such as a rate of fibrosis or a change in a patient's condition after the tubes 11-13 of the implant device for an eye disease are inserted into the anterior chamber 1 of the eyeball.

Alternatively, according to an embodiment, the tubes 11-13 and the implant body may be coupled to each other or integrally formed with each other and may be placed together into the eyeball through the exfoliated conjunctival tissue or Tennon's tissue 4 of the eyeball. That is, the implant body may be pre-coupled to the tubes 11-13 before the implant device is placed in the eyeball, or the tubes 11-13 may be first placed in the eyeball and then may be coupled to the implant body, according to clinical needs or circumstances.

When the implant device for an eye disease is inserted into the eyeball, the aqueous humor generated in the anterior chamber may flow through the tubes 11-13 of the implant device for an eye disease, thereby draining the aqueous humor from the anterior chamber and lowering intraocular pressure. When the implant body is coupled to the tubes 11-13, the aqueous humor drained from the anterior chamber may be temporarily accommodated in the implant body. When the amount of the aqueous humor accommodated in the implant body exceeds a certain amount, the accommodated aqueous humor may be drained to the conjunctival tissue or Tennon's tissue 4 through the rear of the implant body, thereby effectively controlling intraocular pressure.

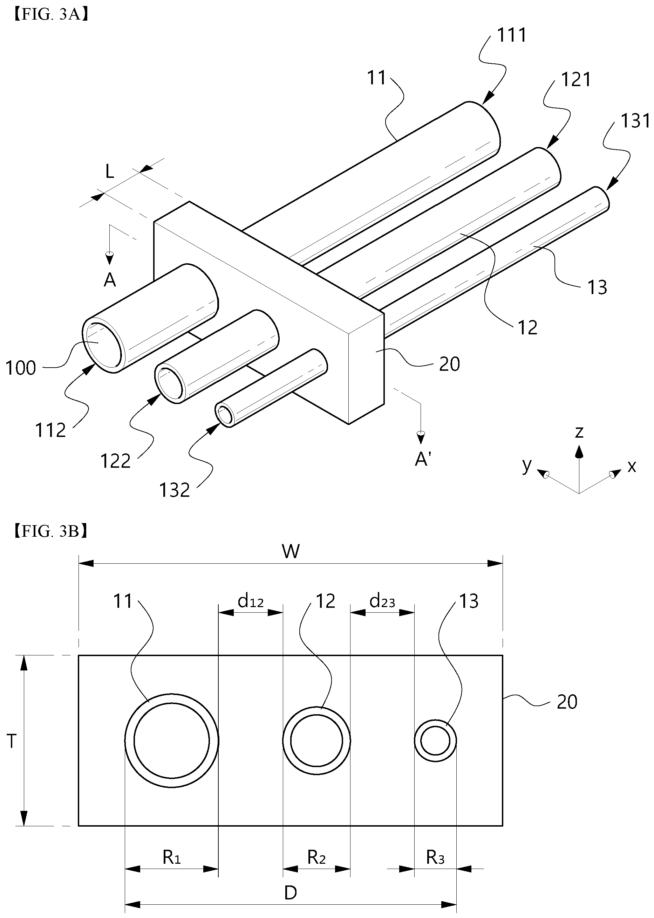

FIG. 3A is a perspective view illustrating an implant device for an eye disease according to an embodiment. FIG. 3B is a cross-sectional view illustrating the implant device for an eye disease taken along line A-A' of FIG. 3A.

Referring to FIGS. 3A and 3B, the implant device for an eye disease according to the present embodiment includes a plurality of tubes 11-13 applicable to MIGS and the connector 20 for coupling the plurality of tubes 11-13. For example, the connector 20 may include one or more holes through which the plurality of tubes 11-13 pass, and the tubes 11-13 may be coupled to the connector 20 by being inserted into the one or more holes formed in the connector 20.

The tubes 11-13 are inserted into an eyeball so that first ends (or distal ends) 111, 121, 131 are located in an anterior chamber of the eyeball and second ends (or proximal ends) 112, 122, 132 opposite to the first ends are located on conjunctival tissue or Tenon's tissue of the eyeball, to drain aqueous humor generated in the anterior chamber of the eyeball to the conjunctival tissue or the Tenon's tissue through hollow portions 100 of the tubes 11-13. In the specification, the distal ends 111, 121, 131 and the proximal ends 112, 122, 132 are defined according to a direction from an operator inserting the implant device, and from among both ends of the tubes 11-13, the proximal ends 112, 122, 132 refer to ends toward the operator and the distal ends 111, 121, 131 refer to ends toward a patient's eye.

In an embodiment, the tubes 11-13 may be formed of a biocompatible material and may be formed of a changeable material. For example, the tubes 11-13 may be formed of silicone or other silicone-based material, a urethane-based material such as PTFE, polycarbonate, or polyurethane (PU), a composite material of a silicone-based material and a polyurethane-based material such as silicone-PU, or a biocompatible metal or alloy.

Also, in an embodiment, the tubes 11-13 may be formed of, but is not limited to, silicone, PTFE, polycarbonate, polyurethane, polyethylene, polypropylene, PMMA, poly(styrene-b-isobutylene-b-styrene) copolymer, polyethersulfone, gelatin, stainless steel, titanium, nitinol, or a combination thereof.

In an embodiment, each of the tubes 11-13 may be formed in a curved shape with a certain curvature in order to prevent damage to the endothelium of a cornea in the eyeball. A front end of the tube may poke and damage the cornea in the anterior chamber of the eyeball in a process of inserting the tube into the anterior chamber of the eyeball according to a size of the eyeball that is different for each patient, a skill level in tube injection, etc. Damage to the cornea may cause complications such as corneal decompensation which require even future corneal transplantation. According to the present embodiment, in order for the tube to naturally move in a curved shape while being inserted into the anterior chamber of the eyeball, each of the tubes 11-13 may be manufactured in a curved shape with a certain curvature corresponding to a curvature of a surface of the eyeball.

The connector 20 for coupling the plurality of tubes 11-13 to each other may have a block or tube shape so that the plurality of tubes 11-13 extend through the connector 20. A length L of the connector 20 along a longitudinal direction (e.g., an x-axis direction of the drawing) of the tubes 11-13 may be appropriately determined to a size such that the connector 20 may fixe and support the tubes 11-13 passing through the connector 20. Also, a width W of the connector 20 along a direction (y-axis direction of the drawing) orthogonal to the longitudinal direction of the tubes 11-13 may be determined to a size larger than a width D of a space occupied by the plurality of tubes 11-13.

That is, the connector 20 surrounds the plurality of tubes 11-13 and has a larger cross-section than the plurality of tubes 11-13. As a result, a part of the connector 20 protrudes in a lateral direction (y-axis direction of the drawing) of the tubes 11-13. As the connector 20 protrudes in the lateral direction of the tubes 11-13, when the tubes 11-13 are pushed toward a sclera of the eyeball, the connector 20 may be caught on the sclera and the tubes 11-13 may be prevented from being completely inserted into the eyeball. Also, a thickness T of the connector 20 in a direction (z-axis direction of the drawing) orthogonal to a plane where the tubes 11-13 are disposed may be determined to be thin enough for the patient not to feel a foreign matter even when the connector 20 is inserted into the conjunctival tissue or Tenon's tissue.

In the embodiment of FIGS. 3A and 3B, the connector 20 is spaced apart from the proximal ends 112, 122, 132 of the tubes 11-13 by a certain distance. However, this is only an example, and in another embodiment, the connector 20 may be in contact with the proximal ends 112, 122, 132 of the tubes 11-13.

In embodiments of the present disclosure, the plurality of tubes 11-13 coupled to each other through the connector 20 are configured to have different aqueous humor drainage characteristics. In the embodiment of FIGS. 3A and 3B, the plurality of tubes 11-13 have different aqueous humor drainage characteristics by having different outer diameters R₁ to R₃. In the present embodiment, the outer diameter R₁ of the tube 11 is larger than the outer diameter R₂ of the tube 12, and also, the outer diameter R₂ of the tube 12 is larger than the outer diameter R₃ of the tube 13. As such, when the plurality of tubes 11-13 are configured to have different outer diameters, even when any one aqueous humor outlet is blocked due to fibrosis of surrounding tissue or foreign material mixing after implant surgery, a situation where all of the plurality of tubes 11-13 are blocked due to the same cause may be prevented.

In FIGS. 3A and 3B, the tubes 11-13 have different outer diameters and thus have different aqueous humor drainage characteristics, assuming that wall thicknesses of the tubes 11-13 are the same. However, when wall thicknesses of the tubes 11-13 are different from each other, even if outer diameters of the tubes 11-13 are the same, inner diameters of the tubes 11-13 are different from each other, so that the tubes 11-13 have different aqueous humor drainage characteristics. Accordingly, the tubes 11-13 may have different aqueous humor drainage characteristics by changing outer diameters and/or inner diameters of the tubes 11-13 so that diameters of the hollow portions 100 of the tubes 11-13, which are substantial sizes of channels through which the aqueous humor flows, are different from each other.

Also, in another embodiment, the tubes 11-13 may have different aqueous humor drainage characteristics by changing lengths, materials, whether a ripcord is used, and/or characteristics of the used ripcord of the tubes 11-13 in addition to the diameters of the hollow portions of the tubes 11-13, which will be described below in detail.

In an embodiment, the holes through which the tubes 11-13 pass into the connector 20 may be spaced apart from each other in the cross-section of the connector 20. As a result, the tubes 11-13 coupled to each other through one connector 20 may be spaced apart from each other in the direction (e.g., the y-axis direction of the drawing) orthogonal to the longitudinal direction thereof. For example, in the embodiment of FIG. 3B, the tube 11 and the tube 12 are spaced apart from each other by an interval d₁₂, and the tube 12 and the tube 13 are spaced apart from each other by an interval d₂₃. However, this is only an example, and in another embodiment, some or all of the plurality of tubes 11-13 may be coupled to the connector 20 while being in close contact with other adjacent tubes.

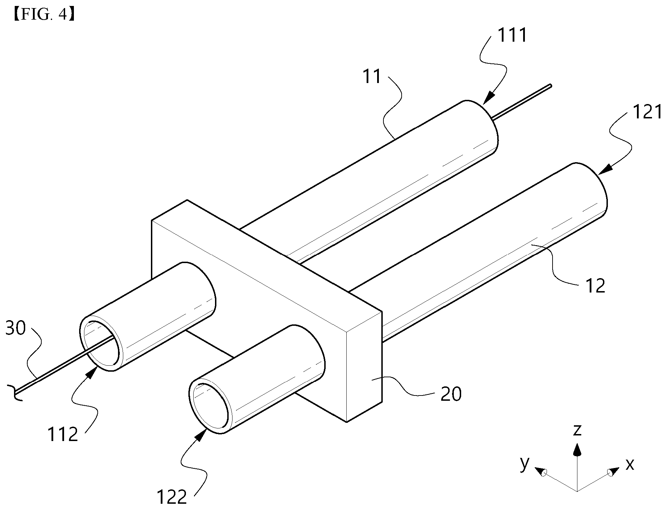

FIG. 4 is a perspective view illustrating an implant device for an eye disease according to another embodiment.

Referring to FIG. 4, the implant device for an eye disease according to the present embodiment further includes the ripcord 30 at least partially inserted into hollow portions of some of the tubes 11, 12. In the present embodiment, the implant device includes the ripcord 30 inserted only into the tube 11 from among the plurality of tubes 11, 12, and the ripcord 30 is not inserted into the other tube 12. The ripcord 30 may be a non-absorbable surgical suture thread, and may be formed of a nylon or prolene material, but the present disclosure is not limited thereto.

The ripcord 30 inserted into the hollow portion of the tube 11 controls pressure formed in an anterior chamber. When the ripcord 30 is thick, a space between an inner wall of the tube 11 and the ripcord 30 becomes narrow and aqueous humor is relatively slowly drained, and thus, pressure formed in the anterior chamber increases. When the ripcord 30 is thin, a space between the inner wall of the tube 11 and the ripcord 30 becomes wide and the aqueous humor is relatively rapidly drained, and thus, pressure formed in the anterior chamber decreases. Accordingly, pressure formed in the anterior chamber may be optimized to be within a certain range, for example, postoperative pressure of about 6 mmHg to about 21 mmHg, through an appropriate configuration of the ripcord 30. However, a preferred numerical range of pressure formed in the anterior chamber is not limited thereto.

Because the ripcord 30 is inserted into only some of the plurality of tubes 11, 12, the plurality of tubes 11, 12 have different aqueous humor drainage characteristics. Although two tubes 11, 12 are illustrated and the ripcord 30 is inserted into only one tube 11 in the drawing, it will be easily understood by one of ordinary skill in the art that the same principle may be applied to an implant device having a larger number of tubes and/or ripcords.

In an embodiment, the ripcord 30 may be manipulated by a clinician to control pressure formed in the anterior chamber. For example, when the ripcord 30 is inserted into the hollow portion of the tube 11 and exposed from a rear end of the tube 11, the clinician may control the amount of aqueous humor drainage by adjusting the ripcord 30 exposed from the rear end of the tube 11. That is, the clinician may appropriately control intraocular pressure according to a patient's condition by using the ripcord 30.

In an embodiment, in order to prevent a user from feeling a foreign matter due to the ripcord 30 exposed to the outside of the tube 11, the ripcord 30 may be formed to gradually decrease in diameter from a point where the ripcord 30 is exposed from the tube 11 or an implant body (not shown) coupled to the rear end of the tube 11.

In the embodiment of FIG. 4, the plurality of tubes 11, 12 have different aqueous humor drainage characteristics by inserting the ripcord 30 into only some of the plurality of tubes 11, 12 constituting the implant device. However, this is only an example, and in another embodiment, ripcords may be respectively inserted into all of the plurality of tubes 11, 12, but the ripcords respectively inserted into the tubes 11, 12 may have different diameters and/or materials. For example, areas of aqueous humor flow channels through the tubes 11, 12 may be different from each other by inserting ripcords having different diameters into the tubes 11, 12. Alternatively, aqueous humor drainage characteristics of the tubes 11, 12 may be substantially different from each other by making materials of ripcords inserted into the tubes 11, 12 different from each other and making degrees of influence of the ripcords on the flow of aqueous humor different from each other.

In surgery of inserting the implant device for an eye disease according to embodiments into the patient's eye, the clinician may insert a ripcord having an appropriate diameter into each tube or a specific tube of the implant device to prevent low intraocular pressure due to excessive drainage of aqueous humor immediately after the surgery. When fibrosis progresses around the tube over time after the implant surgery, the clinician may remove the ripcord at an appropriate time to maintain the effect of lowering intraocular pressure through the implant device for a longer period of time. In this case, a diameter of the ripcord may be appropriately determined according to a length of the tube constituting the implant device, an inner diameter of the tube, and a degree of high intraocular pressure of the patient undergoing the implant surgery.

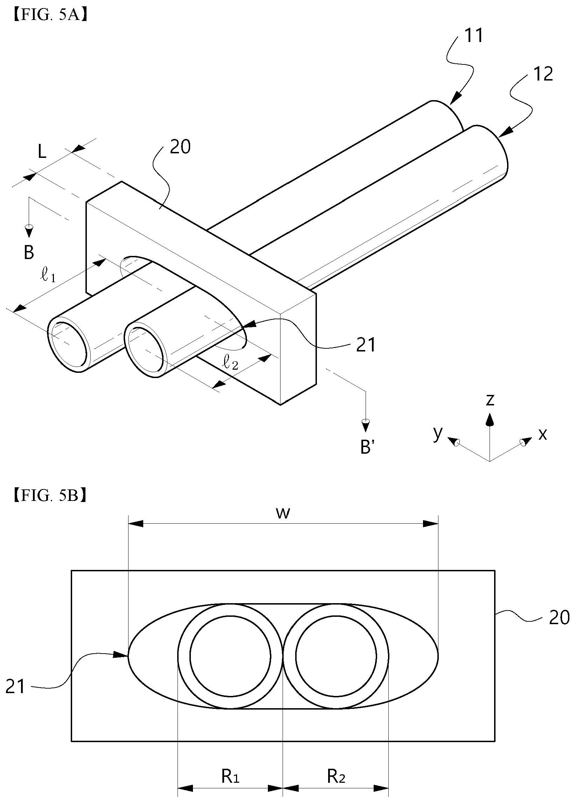

In the embodiments described with reference to FIGS. 1 to 4, a plurality of tubes have different aqueous humor drainage characteristics through physical characteristics of the plurality of tubes themselves included in the implant device or the presence or characteristics of a ripcord inserted into each tube. In another embodiment, a situation where all of the plurality of tubes are blocked due to the same cause may be prevented by making aqueous humor drainage positions (i.e., positions of proximal ends of the tubes) through the plurality of tubes on ocular tissue different from each other, which will be described in detail with reference to FIGS. 5A to 6B.

FIG. 5A is a perspective view illustrating an implant device for an eye disease according to still another embodiment. FIG. 5B is a cross-sectional view illustrating the implant device for an eye disease taken along line B-B' of FIG. 5A.

Referring to FIGS. 5A and 5B, in the present embodiment, the implant device for an eye disease includes a plurality of tubes 11, 12 having different lengths and the connector 20 for coupling the plurality of tubes 11, 12. The plurality of tubes 11, 12 have different lengths, and may be coupled to the connector 20 so that positions of proximal ends of the tubes 11, 12 (i.e., aqueous humor drainage positions) through which aqueous humor is drained are different from each other. In this case, positions of distal ends of the tubes 11, 12 may be the same or different from each other. For example, when a distance from the connector 20 to the proximal end of the tube 11 in an insertion direction (e.g., the x-axis direction of the drawing) of the tubes 11, 12 is l₁, a distance from the connector 20 to the proximal end of the other tube 12 may be l₂ smaller than l₁.

Because a point where the proximal end of each of the tubes 11, 12 is located is an aqueous humor drainage position, aqueous humor drainage positions through the tubes 11, 12 are different from each other by making distances from the connector 20 to the proximal ends of the tubes 11, 12 different from each other. Accordingly, even when fibrosis occurs at a specific point of surrounding tissue after implant surgery and one tube is blocked, another tube whose aqueous humor drainage position is different is not blocked, thereby minimizing side effects due to the blockage of the tube.

Although the implant device includes two tubes 11, 12 in the drawing, it will be easily understood by one of ordinary skill in the art that the same principle may be applied to an implant device including three or more tubes.

In an embodiment, the connector 20 includes a hole 21 through which the plurality of tubes 11, 12 pass. In the present embodiment, one hole 21 through which all of the plurality of tubes 11, 12 may pass is formed in the connector 20, and the plurality of tubes 11, 12 extend through the hole 21 in parallel with each other. In order for the plurality of tubes 11, 12 to pass, the hole 21 may extend in a direction (the y-axis direction of the drawing) orthogonal to a longitudinal direction of the tubes 11, 12. For example, a width w of the hole 21 may be at least greater than a sum (R₁ + R₂) of diameters of the plurality of tubes 11, 12 inserted into the hole 21.

In FIGS. 5A and 5B, aqueous humor drainage positions through the tubes 11, 12 are different from each other by making lengths of the tubes 11, 12 constituting the implant device different from each other. However, in another embodiment, a plurality of tubes having the same shape may be bused, but aqueous humor drainage positions through tubes may be different from each other by adjusting an arrangement or curvatures of the tubes.

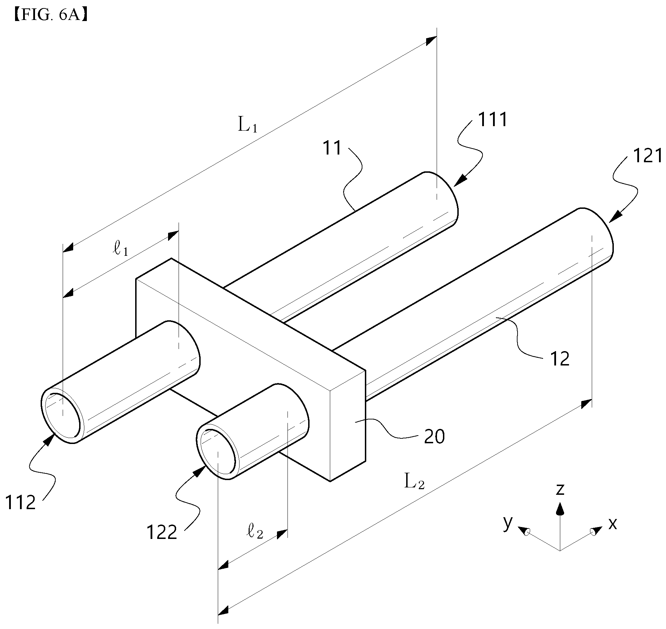

For example, referring to FIG. 6A, in an embodiment, the implant device for an eye disease may include a plurality of tubes 11, 12 having the same physical shape, but positions where the tubes 11, 12 are connected to the connector 20 may be different from each other to have an offset between ends of the tubes 11, 12. That is, in FIG. 6A, a length L₁ of the first tube 11 and a length L₂ of the second tube 12 are the same, but the second tube 12 may be disposed closer to an eyeball than the first tube 11, so that distances l₁, l₂ from the connector 20 to proximal ends of the tubes 11, 12 are different from each other, and thus, aqueous humor drainage positions through the tubes 11, 12 are different from each other.

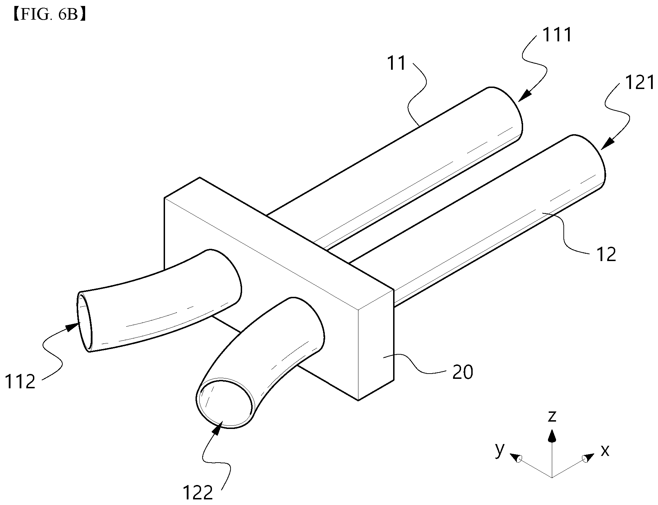

Also, referring to FIG. 6B, in an embodiment, the implant device for an eye disease may include a plurality of tubes 11, 12 having the same physical shape, but the tubes 11, 12 may be bent in different directions so that proximal ends 112, 122 of the tubes 11, 12 are located at different points on ocular tissue. For example, although the plurality of tubes 11, 12 are commonly coupled to the connector 20, the tubes 11, 12 may be bent in a Y-shape so that an interval between the tubes 11, 12 increases away from the connector 20, and thus, aqueous humor drainage positions through the tubes 11, 12 may be different from each other.

Although aqueous humor drainage positions through the tubes 11, 12 are different from each other by adjusting positions and curvatures of the plurality of tubes 11, 12 having the same shape in FIGS. 6A and 6B, it will be understood by one of ordinary skill in the art that the same principle may be applied to a case where a plurality of tubes have different physical shapes (e.g., lengths, materials, diameters of hollow portions, whether ripcords are inserted, and diameters and/or materials of the ripcords).

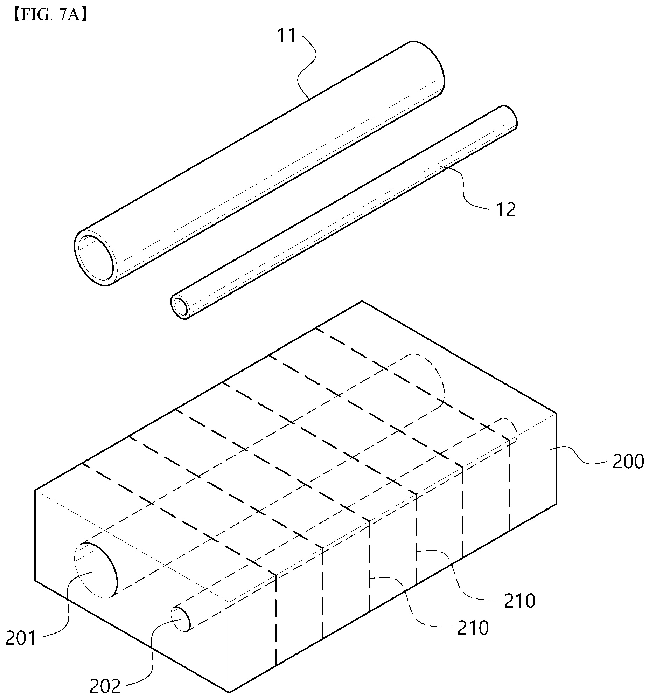

FIG. 7A is a conceptual view for describing a process of manufacturing an implant device for an eye disease according to an embodiment.

Referring to FIG. 7A, in order to manufacture an implant device according to the present embodiment, a body 200 to be formed as a connector and a plurality of tubes 11, 12 may be formed. The body 200 may be formed of silicone or another appropriate material, and may be formed of the same material as the tubes 11, 12. When the tubes 11, 12 are formed of silicone, the tubes 11, 12 may be manufactured by using a drawing method. In order to couple the drawn tubes 11, 12 and fix positions of the tubes 11, 12, a connector may be additionally manufactured separately from the tubes 11, 12 and the tubes 11, 12 may be assembled to the connector.

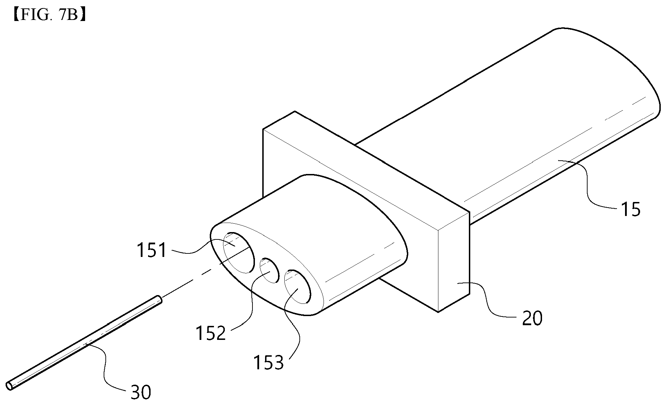

In order to manufacture the connector, the body 200 of the connector having a block shape extending in one direction may be formed by drawing of silicone or the like. Next, one or more holes 201, 202 through which the tubes 11, 12 pass may be formed in the body 200. In an embodiment, a plurality of connectors may be manufactured at once by cutting the body 200 in which the holes 201, 202 are formed along a longitudinal direction. A cutting line 210 shown in FIG. 7 is an exemplary cutting line for dividing the body 200 into a plurality of pieces along the longitudinal direction of the body 200. Finally, the implant device for an eye disease according to embodiments may be manufactured by inserting the plurality of tubes 11, 12 into the one or more holes 201, 202 of the body 200.

Although two holes 201, 202 having different diameters are formed in the body 20 to allow two tubes 11, 12 having different diameters to pass therethrough in the present drawing, this is only an example, and when aqueous humor drainage characteristics of the tubes 11, 12 are different from each other through features other than diameters or when the tubes 11, 12 having the same specification are used but are coupled to the body 200 so that drainage positions are different from each other, diameters of holes formed in the body 200 may be the same. In another embodiment, a single hole passing through the body 200 may be formed, but the single hole may have a size that allows the plurality of tubes 11, 12 to be inserted thereinto.

Although a process of manufacturing an implant device for an eye disease has been described assuming that two tubes 11, 12 are used in the embodiment of FIG. 7A, it will be easily understood by one of ordinary skill in the art that the sample principle may be applied to a case where an implant device using three or more tubes is manufactured.

In another embodiment, the implant device for an eye disease may be integrally formed by using injection molding or the like. That is, in the manufacture of the implant device for an eye disease according to the embodiments described with reference to FIGS. 1 to 6B, the plurality of tubes 11-13 having different aqueous humor drainage characteristics may be integrally formed as one tube body, and/or the plurality of tubes 11-13 and the connector 20 coupled to the plurality of tubes 11-13 may be integrally formed with each other.

FIG. 7B illustrates an embodiment of an implant device for an eye disease that is integrally formed by using injection molding or the like.

Referring to FIG. 7B, in the present embodiment, a tube body 15 has a shape in which a plurality of tubes defined by hollow portions 151-153 are integrally formed with each other. In other words, the tube body 15 is one tube member formed to have the plurality of hollow portions 151-153 having different aqueous humor drainage characteristics. The plurality of hollow portions 151-153 may have different diameters to have different aqueous humor drainage characteristics. Also, the ripcord 30 for adjusting the amount of aqueous humor drainage through the hollow portions 151-153 may be inserted into the tube body 15.

The connector 20 may be coupled to the tube body 15 so that the tube body 15 extends in a direction different from a longitudinal direction of the connector 20 (e.g., a direction orthogonal to the longitudinal direction of the connector 20). The tube body 15 and the connector 20 may be separately formed and then may be coupled to each other, or the tube body 15 and the connector 20 may be integrally formed from the beginning by using a method such as injection molding, to manufacture the implant device for an eye disease. That is, when the implant device for an eye disease is integrally formed, the tube body 15 and the connector 20 may refer to different portions of a single molded product formed of silicone or another material.

A shape of the implant device for an eye disease shown in FIG. 7B is only an example, and a manufacturing method of integrally forming a plurality of tubes with each other and/or integrally forming a plurality of tubes and a connector with each other may be applied to embodiments illustrated in other drawings of the specification.

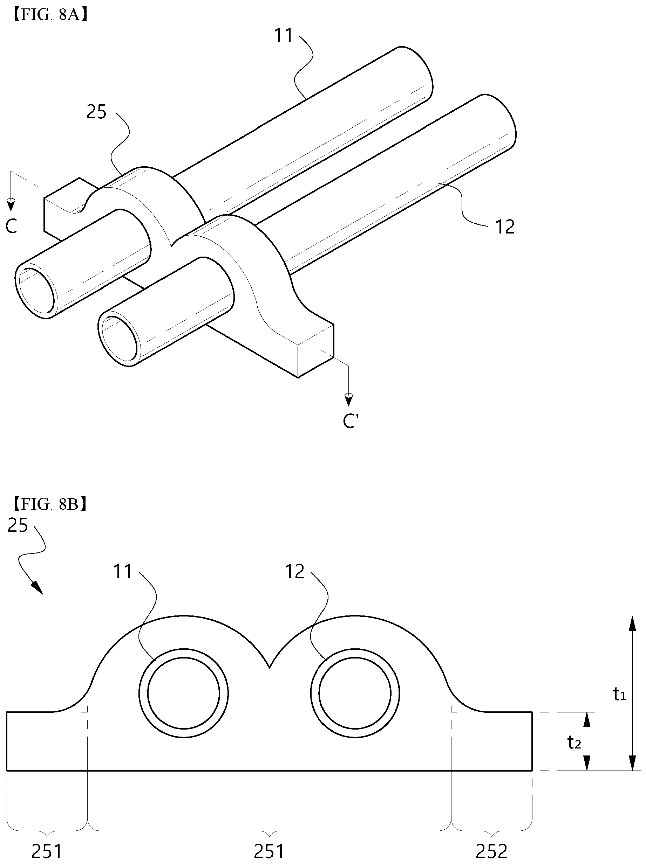

FIG. 8A is a perspective view illustrating an implant device for an eye disease according to still another embodiment. FIG. 8B is a cross-sectional view illustrating the implant device for an eye disease taken along line C-C' of FIG. 8A.

Referring to FIGS. 8A and 8B, in the present embodiment, a cross-section of a connector 25 may have an uneven shape all or part of which has a curved shape corresponding to shapes of the tubes 11, 12. In the embodiment of the drawing, the connector 25 has a bottom surface having a flat plate shape and a top surface having a cylindrical shape corresponding to shapes of the tubes 11, 12.

In an embodiment, the connector 25 may include a first portion 251 through which the plurality of tubes 11, 12 pass and a second portion 252 extending from the first portion 251 in a lateral direction of the tubes 11, 12. That is, the second portion 252 may protrude in the lateral direction of the tubes 11, 12, in a wing-like shape. In an embodiment, a thickness t₂ of the second portion 252 may be less than a thickness t₁ of the first portion. In still another embodiment, the second portion 252 may have a tapered shape in which the thickness t₂ of the second portion 252 increases or decreases away from side surfaces of the tubes 11, 12.

In the present embodiment, the second portion 252 having a wing shape may be used to fix a position so that the implant device is well seated in an eyeball. For example, the implant device may be fixed to tissue when the second portion 252 is coupled to surrounding tissue through fibrosis after implant surgery. Also, because the connector 25 includes the second portion 252 having a wing shape to further increase a cross-sectional size of the implant device, the tubes 11, 12 may be prevented from falling into the eyeball after the implant surgery. Furthermore, when necessary, the second portion 252 of the connector 25 may be fixed to the eyeball by using a suture thread.

Although the second portion 252 having a wing shape is applied to the connector 25 having an uneven cross-section in FIGS. 8A and 8B, the second portion having a wing shape may be applied to a connector having any of other different cross-sectional shapes.

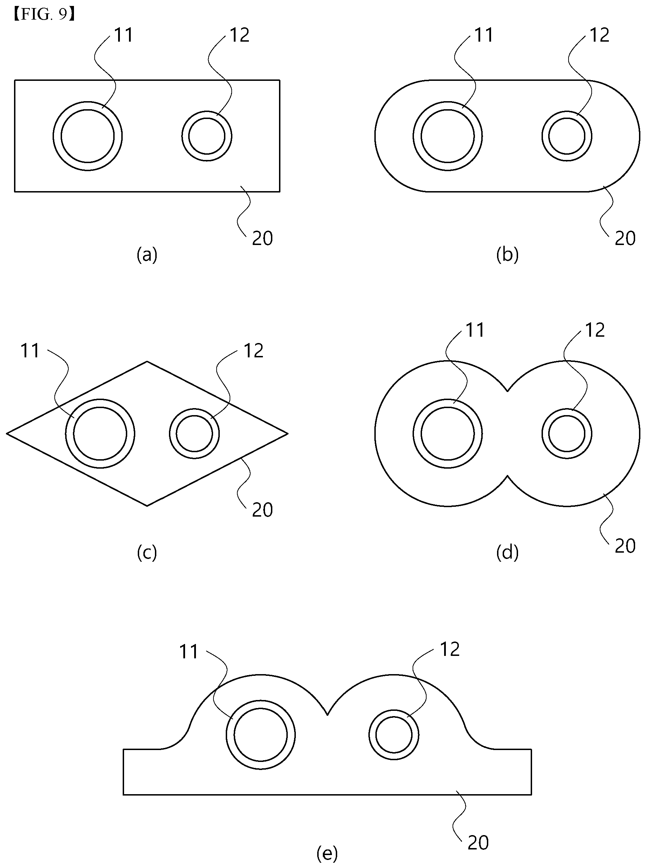

FIG. 9 is cross-sectional views illustrating various cross-sectional shapes of a connector in an implant device for an eye disease according to embodiments.

In embodiments of the present disclosure, a connector may have a quadrangular cross-section as shown in (a) of FIG. 9, may have an elliptical cross-section as shown in (b) of FIG. 9, or may have a rhombus cross-section as shown in (c) of FIG. 9. Also, the connector may have an uneven shape with a curved cross-section corresponding to shapes of the tubes 11, 12 as shown in (d) and (e) of FIG. 9, or may have any of other cross-sections not shown in the drawings of the specification. Furthermore, the connector may have a wing protruding in a lateral direction as shown in (e) of FIG. 9, and the wing may be applied to the connectors having other cross-sections shown in (a) to (d) of FIG. 9.

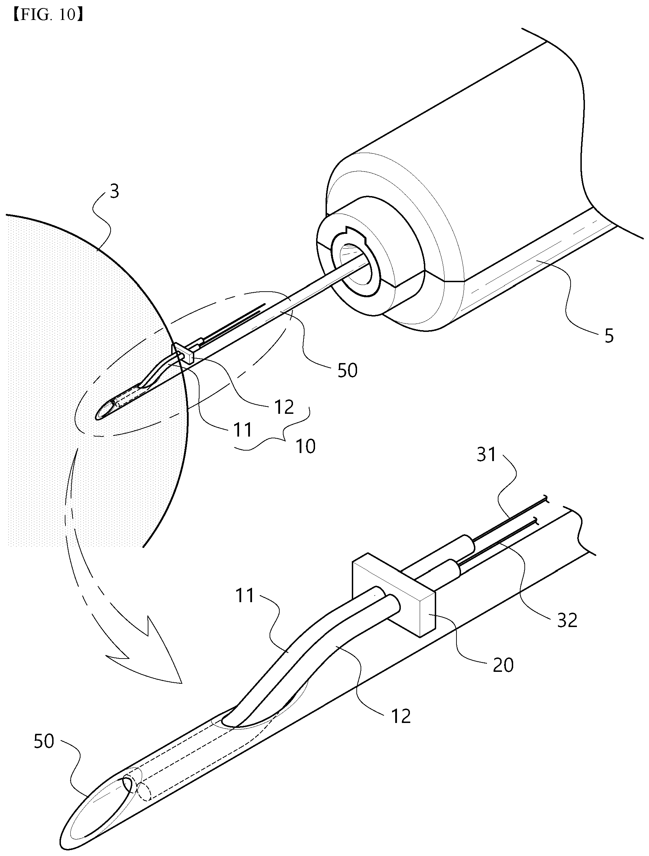

FIG. 10 is a conceptual view illustrating a process of inserting an implant device for an eye disease according to an embodiment into an eyeball by using an injector.

Referring to FIG. 10, the implant device for an eye disease according to embodiments may be injected into an eyeball through an injector 5 including a needle 50 so that ends of the tubes 11, 12 are inserted into an anterior chamber through the sclera 3 of the eyeball as shown in FIGS. 1 and 2. Here, the injector 5 refers to an arbitrary instrument used to insert the implant device into the eyeball by mechanically moving the needle 50 or the injector 5 forward in a state where the implant device is accommodated in the needle 50 or is fixed to the needle 50 by using another method.

For example, in a state where the implant device is loaded in the needle 50, an operator may move the entire injector 5 forward so that ends of the tubes 11, 12 of the implant device are inserted into the anterior chamber through the sclera 3, and then with the implant device in the inserted position, the operator may move only the needle 50 backward through an arbitrary mechanism provided in the injector 5 or move the entire injector 5 backward so that the implant device is inserted into the eyeball. Because the injector 5 for inserting the implant device may be any of various types well known or to be developed in the future, a detailed description of the injector 5 will be omitted in the specification to clarify the gist of the present disclosure.

Because the implant device for an eye disease according to embodiments includes the connector 20 having a cross-section larger than diameters of the tubes 11, 12, the connector 20 may be inserted into the needle 50 of the injector 5 together with the tubes 11, 12, or the implant device may be inserted into a patient's eye in a state where the connector 20 is located outside the needle 50.

For example, in an embodiment, the connector 20 has a cross-section larger than diameters of the tubes 11, 12 but may be rolled or folded because the connector 20 is formed of a flexible material. Accordingly, the connector 20 may be inserted into the needle 50 together with the tubes 11, 12 while being rolled or folded to be in close contact with surfaces of the tubes 11, 12, and when the tubes 11, 12 reach an insertion position, the connector 20 may be unfolded while exiting the needle 50 together with the tubes 11, 12. Alternatively, in a state where the connector 20 is not inserted into the needle 50 and only front ends of the tubes 11, 12 not coupled to the connector 20 are inserted into the needle 50 or fixed to the needle 50, the implant device may be placed in the eyeball in such a way that the needle 50 pulls the connector 20 together through the front ends of the tubes 11, 12.

However, a method of arranging the tubes 11, 12 and the connector 20 is not limited to the examples described in the specification. Any other clinical method may be used. For example, the connector 20 may be coupled to the tubes 11, 12 through an additional procedure in a state where only the tubes 11, 12 are inserted into the eyeball by using the injector 5. The operator may first insert the tubes 11, 12 of the implant device into the patient's sclera, and then may couple the tubes 11, 12 to the connector 20 by attaching the connector 20 to ends of the tubes 11, 12 exposed to the outside of the sclera.

As described above, in the implant device according to embodiments, the tubes 11, 12 are for MICS and usually have micrometer-level diameters, and thus, there is a possibility that the tubes 11, 12 may be pushed into the sclera 3 due to impact or other causes even after the tubes 11, 12 are inserted into the sclera. However, according to embodiments of the present disclosure, because the connector 20 having a cross-section larger than diameters of the tubes 11, 12 is coupled to the tubes 11, 12, even when the tubes 11, 12 move toward the sclera 3, the connector 20 may be caught on the sclera 3 and the tubes 11, 12 may be prevented from being completely inserted into the sclera 3.

In the implant device injected through the injector 5 shown in FIG. 10, the plurality of tubes 11, 12 having different lengths are inserted side by side into the connector 20 while being in close contact with each other as shown in FIG. 5A. However, this is only an example, and it will be easily understood by one of ordinary skill in the art that the implant device for an eye disease according to other embodiments illustrated in the attached drawings of the specification or described through the specification may be placed in the eyeball by using the injector 5 by using the above-described method.

The above description of the present disclosure is provided for illustration, and it will be understood by one of ordinary skill in the art that various changes in form and details may be readily made therein without departing from essential features and the scope of the present disclosure. Accordingly, the above embodiments are examples only in all aspects and are not limited. For example, each component described as a single type may be executed in a distributed manner, and components described as a distributed type may be executed in a combined manner.

The scope of the present disclosure is indicated by the claims rather than by the detailed description of the present disclosure, and it should be understood that the claims and all modifications or modified forms drawn from the concept and scope of the claims and equivalents are included in the scope of the present disclosure.

### [Industrial Applicability]

The present disclosure relates to an implant device for an eye disease and a method of manufacturing the same, and more particularly, to an implant device for an eye disease configured to prevent loss of an aqueous humor drainage function due to fibrosis or foreign material mixing after implant surgery by connecting a plurality of tubes having different aqueous humor drainage characteristics (drainage amount, drainage rate, etc.) and/or different drainage positions through a connector.

## Claims

1. An implant device for an eye disease to be inserted into an eyeball, the implant device for an eye disease comprising:
a plurality of tubes each having one end to be inserted into an anterior chamber of the eyeball and having a hollow portion through which aqueous humor is drained; and
a connector configured to couple the plurality of tubes,
wherein the connector is coupled to the plurality of tubes so that the plurality of tubes extend through the connector.

2. The implant device for an eye disease according to claim 1, wherein the plurality of tubes are different from each other in at least one of a length, a material, and a diameter of a hollow portion, and are different from each other in at least one of aqueous humor drainage characteristics or an aqueous humor drainage position.

3. The implant device for an eye disease according to claim 2, wherein each of the plurality of tubes comprises a first end to be inserted into the anterior chamber of the eyeball and a second end opposite to the first end,
wherein the second ends of the plurality of tubes are located at different positions from the connector.

4. The implant device for an eye disease according to claim 1, further comprising a ripcord inserted into the hollow portions of some of the plurality of tubes.

5. The implant device for an eye disease according to claim 1, further comprising a plurality of ripcords respectively inserted into the hollow portions of the plurality of tubes,
wherein the plurality of ripcords are different from each other in at least one of a diameter and a material.

6. The implant device for an eye disease according to claim 1, wherein the connector comprises a plurality of holes through which the plurality of tubes respectively pass, the plurality of holes being spaced apart from each other in a cross-section of the connector.

7. The implant device for an eye disease according to claim 1, wherein the connector comprises a hole extending in a direction orthogonal to a longitudinal direction of the plurality of tubes so that the plurality of tubes pass through the hole.

8. The implant device for an eye disease according to claim 1, wherein the connector has a cross-section larger than a width of the plurality of tubes.

9. The implant device for an eye disease according to claim 8, wherein the connector comprises:
a first portion through which the plurality of tubes pass; and
a second portion extending from the first portion in a direction different from an extending direction of the plurality of tubes, the second portion protruding in a lateral direction of the plurality of tubes.

10. The implant device for an eye disease according to claim 9, wherein a thickness of the second portion is less than a thickness of the first portion.

11. The implant device for an eye disease according to claim 1, wherein the plurality of tubes are integrally formed with each other.

12. The implant device for an eye disease according to claim 1, wherein the plurality of tubes and the connector are integrally formed with each other.

13. A method of manufacturing an implant device for an eye disease, the method comprising:
forming a plurality of tubes each having a hollow portion through which aqueous humor is drained;
forming a body of a connector extending in one direction;
forming, in the body, one or multiple holes through which the plurality of tubes pass; and
inserting the plurality of tubes into the hole of the body,
wherein the plurality of tubes are different from each other in at least one of aqueous humor drainage characteristics or an aqueous humor drainage position.

14. A method of manufacturing an implant device for an eye disease, the method comprising forming a tube body comprising a plurality of tubes each having a hollow portion through which aqueous humor is drained and a connector integrally connected to the plurality of tubes,
wherein the connector extends in one direction and is coupled to the plurality of tubes so that the plurality of tubes extend in a direction different from a longitudinal direction of the connector,
wherein the plurality of tubes are different from each other in at least one of aqueous humor drainage characteristics or an aqueous humor drainage position.
